# EUROPEAN PATENT SPECIFICATION

(11) **EP 3 132 262 B1**
(45) Date of publication and mention of the grant of the patent: **13.12.2017**
(21) Application number: 15716773.5
(22) Date of filing: 14.04.2015
(51) Int. Cl.: G01N 33/49

(54) **GAMMA IRRADIATION STABILIZED DEXTRAN SOLUTIONS AND METHODS OF USE**
GAMMASTRAHLUNGSSTABILISIERTE DEXTRANLÖSUNGEN UND VERFAHREN ZUR VERWENDUNG
SOLUTIONS DE DEXTRANE STABILISÉES PAR IRRADIATION GAMMA ET PROCÉDÉS D'UTILISATION

(30) Priority: 16.04.2014 US 201414254152
(43) Date of publication of application: 22.02.2017
(73) Proprietor: General Electric Company, Schenectady, NY 12345 (US)
(72) Inventor: MCCLOSKEY, Patrick, Joseph, Niskayuna, New York 12309 (US); PICKETT, James, Edward, Niskayuna, New York 12304 (US); GODDARD, Gregory, Daryll, Niskayuna, New York 12309 (US); SMITH, Reginald, Donovan, Niskayuna, New York 12309 (US); BAJAJ, Anshika, Niskayuna, New York 12309 (US)
(74) Representative: Aldenbäck, Ulla Christina
(86) International application number: PCT/EP2015/058015
(87) International publication number: WO 2015/158683

(56) References cited:
- WO-A1-00/60351
- US-A1- 2003 109 494
- US-A1- 2009 081 689
- PRIBUSH A ET AL: "The mechanism of the dextran-induced red blood cell aggregation", EUROPEAN BIOPHYSICS JOURNAL ; WITH BIOPHYSICS LETTERS, SPRINGER, BERLIN, DE, vol. 36, no. 2, 8 November 2006 (2006-11-08), pages 85-94, XP019472433, ISSN: 1432-1017
- BJÖRK V O ET AL: "The effects of ascorbic acid and mannitol on induced red blood cell aggregation.", ACTA CHIRURGICA SCANDINAVICA. SUPPLEMENTUM 1965, vol. 356, 1965, pages 95-99, XP008176697, ISSN: 0301-1860

## Description

### BACKGROUND

Separation of red blood cells (RBC) from whole blood is commonly required prior to analysis or therapeutic use of less abundant cells, such as white blood cells or stem cells. Many conventional blood cell isolation procedures require preliminary red blood cell depletion and sample volume reduction. These steps are commonly performed in long-term cell banking and regenerative medicinal applications, where a maximal yield of nucleated blood cells is desired in a reduced volume for direct transplantation or storage for future use.

Often these methods utilize dextran as an aggregant to enhance the sedimentation of red blood cells from whole blood. A critical part of the manufacturing process is the sterilization of the kit which is accomplished by exposure to gamma irradiation at a dose between 20 and 50 kGy, sufficient to ensure sterilization. Unfortunately, dextran in water is unstable to gamma irradiation resulting in severe molecular weight decomposition of the dextran. For example, 3,300 kD Mw dextran will decompose to less than 20 kD on exposure to only a 20 kGy dose of gamma irradiation. Furthermore, RBC sedimentation enhancement performance of the added dextran is a function of its molecular weight and is ineffective below 200 kD molecular weight. As a result the dextran solution must be sterilized separately by autoclaving or filtering the solution then reassembling with the gamma sterilized kit adding cost and potential contamination during manufacturing. US 2003/109494 discloses a solution comprising Dextran (2 grams, average molecular weight 15,000) dissolved in water (100 ml) at pH 5.2 to which Ascorbic acid (2 grams) was added.

US2009/081689 discloses a method of aggregating cells in a sample containing red blood cells involving adding a dextran solution with dextrans of over 500kD.

As such there is a need for gamma stabilized dextran solutions which will allow incorporation of the dextran more directly into the handling and manufacturing process to insure stability but also reduce handling errors and cost.

### BRIEF DESCRIPTION

In general, the methods and kits of the invention provide gamma stable dextran solutions, which can be sterilized through gamma irradiation while maintaining a sufficient molecular weight to subsequently act as a red blood cell (RBC) aggregant. This increases the efficiency of blood separation as the dextran solution may be incorporated directly into the handling and manufacturing process

One embodiment provides an aqueous dextran solution, stable to gamma irradiation. The solutions comprising 1 to 10 wt /v % of dextran, where the dextran has an initial average molecular weight greater than 500kD, and 2.0 to 20.0 wt% ascorbic acid or its mineral salt to the dextran.

In another embodiment, a kit is provided for producing a gamma stabilized aqueous dextran solution comprising dextran having an initial average molecular weight greater than 500kD, and 2.0 to 20.0 wt% ascorbic acid or its mineral salt to the dextran.

In another embodiment a method provides for adding the gamma stabilized aqueous solution to a blood sample (peripheral blood, cord blood), resulting in increased red blood cells (RBC) aggregation and sedimentation while recovering a large percentage of the total nucleated cells (TNC). The method comprises the steps of subjecting a dextran solution comprising ascorbic acid or a mineral salt of ascorbic acid to gamma radiation, adding to the blood sample, incubating to aggregate RBCs, and recover TNCs.

### FIGURES

These and other features, aspects, and advantages of the present invention will become better understood when the following detailed description is read with reference to the accompanying figure.

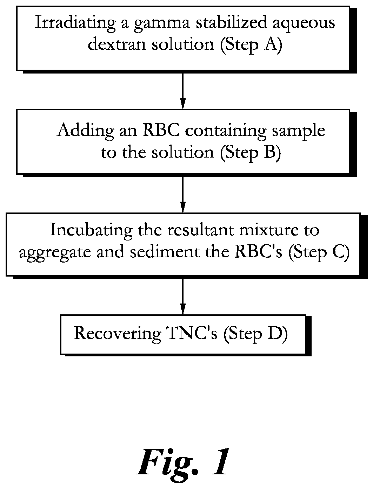

FIG. 1 is flow diagram of the process to sediment cells using a gamma stabilized dextran solution which undergoes sterilization through exposure to gamma irradiation.

### DETAILED DESCRIPTION

The following detailed description is exemplary and not intended to limit the invention of the application and uses of the invention. Furthermore, there is no intention to be limited by any theory presented in the preceding background of the invention on the following detailed description. To more clearly and concisely describe and point out the subject matter of the claimed invention, the following definitions are provided for specific terms that are used in the following description and the claims appended hereto.

Unless otherwise indicated, the article "a" refers to one or more than one of the word modified by the article "a." Unless otherwise indicated, all numbers expressing quantities of ingredients, properties such as molecular weight, reaction conditions, so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

"Dextran" refers to polysaccharides with molecular weights ≥1000 Dalton (Da), which have a linear backbone of α-linked D-glucopyranosyl repeating units and typically have a molecular weight ranging from 3,000Da to 2,000,000Da. It is often classified according to molecular weight. For example dextran 500 refers to an average molecular mass of 500 kDa. Dextran 1230 refers to an average molecular mass of 1230kDa.

"Kit" is referred to herein as one or more reactants or additives necessary for a given assay, test, or process. The kit may also include a set of directions to use the reactants or additives present in the kit, any buffers necessary to maintain processing conditions or other optional materials for using. In certain cases the kit may contain premeasured amounts of the reactants or additives for a given assay, test, or process.

In certain embodiments a gamma stabilized dextran solution is provided, the solution comprising of an aqueous solution of dextran, ascorbic acid or a mineral salt of ascorbic acid. In certain embodiments, the dextran solution is approximately 1 to 10 wt% of dextran in an aqueous solution. Preferably the dextran is 1 to 5wt % and more preferable 2 to 4wt % dextran in an aqueous solution. In certain embodiments the red blood cell sedimentation enhancement performance of added dextran is a function of molecular weight and it is ineffective below 200kD molecular weight. As such in certain embodiments, after gamma irradiation the dextran has a molecular weight greater than approximately 200kD. In preferred embodiments, the molecular weight is in a range of approximately 200-800 kD and most preferred the dextran has molecular weight in a range of approximately 400-600 kD after gamma irradiation. In certain embodiments the dose of gamma radiation is between 20 and 50kGy, and more preferably at a dose between 25 and 45 kGy.

In certain embodiments, the ascorbic acid is a mineral salt including, but not limited to sodium ascorbate, calcium ascorbate, potassium ascorbate, magnesium ascorbate, zinc ascorbate or a combination thereof. In certain embodiments the mineral salt is sodium ascorbate.

In certain embodiments the ascorbic acid or its mineral salt added from approximately 2 to 20 wt% to an aqueous dextran in preferred embodiments from 4 to 15 wt %, and more preferable from 4 to 10 wt %. In certain other embodiments the mineral salt is added directly to dextran at approximately 2 to 20 wt%, in preferred embodiments from 4 to 15 wt %, and more preferable from 4 to 10 wt%. The ascorbic acid or its mineral salt may act as a radiation stabilizer, conserving the dextran molecular weight at a level sufficient to act as an aggregant to enhance the sedimentation of RBC.

In certain embodiments, the gamma stabilized dextran solution may further comprise buffers. The buffer comprises organic or inorganic salts that maintain a pH of 4.0 to 8.0 such as such as phosphate buffered saline (PBS). The solution may also comprise other non-toxic enhancers such as, sodium citrate, sodium succinate and combinations thereof. The use of non-toxic enhancers, the methods of aggregating blood cells and its use in connection with the system and methods are further described in U.S. Patent Application, Serial No. 12/325672, entitled "SYSTEMS AND METHODS FOR PROCESSING COMPLEX BIOLOGICAL MATERIALS'.

As shown in FIG. 1 in certain embodiments a method to sediment cells improve the resulting recovery of an increased percentage of total nucleated cells (TNCs) from a sample comprising red blood cells (RBC), where the method comprises the steps of adding the RBC sample to the aforementioned gamma stabilized dextran solution comprising ascorbic acid or a mineral salt of ascorbic acid to (Step A). In certain embodiments, the method further comprises incubation of the sample to aggregate and sediment the plurality of RBCs (Step C) and/or eventual recovering the TNC (Step D). For example, Steps C and D are commonly performed in long-term cell banking and regenerative medicinal applications, where a maximal yield of nucleated blood cells is desired in a reduced volume for direct transplantation or storage for future use (Step D). In certain embodiments, the method of recovering the TNC may comprise concentration of a liquid phase prior to collection. The liquid phase comprises plasma and dextran and thus concentration may be accomplished through a number of methods including centrifugation, membrane filtration, or a combination of methods.

In certain embodiments the sample comprising the RBC is whole blood, in certain other embodiments the sample comprising the RBC is a blood component, such as but not limited to isolated blood fraction including bone marrow and mobilized peripheral blood.

In certain embodiments, a kit is provided comprising the reactants and additives necessary for producing a gamma stabilized dextran solution. In certain embodiments, the kit comprises of dextran, abscorbic acid or a mineral salt of ascorbic acid. In certain embodiments, the dextran has a molecular weight (MW) that is sufficient to maintain a MW greater than approximately 200kD after exposure to gamma radiation. In certain embodiments, the initial molecular weight of dextran is greater than 500 kD, in preferred embodiments greater than 750kD and most preferred greater than 1000kD. In certain other embodiments, the initial molecular weight of dextran is between 1000kD and 2000kD, in preferred embodiments the initial molecular weight of dextran is approximately 1000-1500kD.

In certain embodiments, the components of the kit are dry mixed in an amount that, when added to an aqueous solution, yields a gamma stabilized dextran solution of approximately 1 to 10 wt% of dextran in an aqueous solution. Preferably the dextran is 1 to 5wt % and more preferable 2 to 4wt % dextran when used in an aqueous solution.

In certain embodiments, the kit may further comprise buffers such as phosphate buffered saline (PBS), saline and or other non-toxic enhancers such as, sodium citrate, sodium succinate and combinations thereof. The additives may be combined in such a way that when added together in an aqueous solution, a gamma stabilized dextran solution is obtained. In certain embodiments, the kit is prepared in such a way that the individual components are provided separately. In other embodiments, the kit is prepared such that components in solid form may be premixed and supplied together. In still other embodiments, the kit is prepared such that certain components are provided in solution.

The methods and kits of the invention to sediment blood cells generally comprise adding the gamma stabilized dextran solution to accelerate RBC sedimentation. For example, in one embodiment, a sample that includes red blood cells is treated by adding an irradiated gamma stabilized dextran solution, followed by incubation of the sample, and eventual recovery of the total nucleated cells (TNCs).

One or more of the methods of recovering a percentage of TNCs from a sample comprising red blood cells comprises adding a gamma stabilized dextran solution which has been irradiated, at a predetermined concentration,, incubation of the sample, and eventually recovering of the total nucleated cells.

### EXAMPLES

Practice of the invention will be more fully understood from the following examples, which are presented herein for illustration only and should not be construed as limiting the invention in any way.

Materials: Human cord blood was used for the experiments. The dextran 1230 used in this example was obtained from GE Healthcare, (Uppsala, Sweden). Ammonium formate and sodium ascorbate are available from Sigma-Aldrich (St. Louis, Missouri). Sodium citrate was obtained from Thermo Fisher Scientific (Waltham, Massachusetts). Pure dextran samples were prepared by dissolving approximately 37.5mM of sodium citrate and 2.25% (w/v) of dextran in 40% (v/v) of saline (Baxter, Deerfield IL) and 31.6 % (v/v) of water for injection (Baxter, IL). Dextran and sodium citrate were allowed to dissolve for 2 hours after which the remaining saline was added to make up the desired volume. All samples were allowed to dissolve for at least 2hr prior to analysis.

Analysis of dextrans was accomplished using Gel Permeation Chromatography in Combination with Multi-Angle Laser Light Scattering.

Static Light Scattering analysis in combination with aqueous phase gel permeation chromatography was achieved using an Agilent 1100 series HPLC in combination with a Wyatt Technologies Dawn EOS Multi-Angle Light Scattering Detector in-line to a Wyatt Optilab DSP Interferometric Refractive index detector. The light scattering detector collects scattered light at 18 angles upstream of the refractive index (DRI) data acquisition. The function of the DRI detector is to provide a concentration term from the RI response of a given analyte with a known refractive index increment value (DN/DC) for use in the first principle calculation equation for molar mass. The chromatography was achieved using an isocratic elution of 2mM ammonium formate (pH 4-5). The molar mass values were obtained using the first principle calculation as derived from the Zimm formalism.

The GPC separation was achieved using 2 Tosoh PW Columns: 1-G6000 and 1-G3000 aqueous SEC columns (7.5x300mm) in series at a flow rate of 1ml min-1 run at ambient temperature as outlined in Table 1.

**Table 1. Method Parameters for Gel Permeation Chromatography in combination with Multi-Angle Laser Light Scattering / Differential Refractive Index Detection of Dextrans.**

| Instrument | Agilent 1100 Series HPLC / Wyatt EOS Multi- Angle Laser Light Scattering Detector w/ Quasi-Elastic LS Accessory (QELS) / Optilab Differential Refractive Index Detector |
|---|---|
| Column | 1-Tosoh G3000 PW (7 x 300mm) and 1-Tosoh G3000 PW (7 x 300mm) |
| Mobile Phase | 2mM Ammonium Formate pH=4 -5 |
| Flow | 1ml / min |
| Temperature | Ambient (28-30C) |
| Injection Volume | 20ul |
| DRI Temperature | 35C |
| DNDC | 0.145 ml/g |
| Gradient Profile | Isocratic Elution |

Table 2 shows the effects of ascorbic acid or its sodium salt added from 1.0 to 10.0 wt% to an aqueous dextran solution followed by exposure to 40 kGy dose of Gamma irradiation (Cobalt 60)

**Table 2. Dextran solutions exposed to 40kGy dose of gamma radiation**

| **Sample #** | **Dextran Mw (kD)** | **% Ascorbate** | **Gamma dose (kGy)** | **Mw after irradiation** |
|---|---|---|---|---|
| 6 | Dextran AB (1,230) | 0 | none | 1,230 |
| 12 | Dextran AB (1,230) | 0 | 40 | 35 |
| 13 | Dextran AB (1,230) | 1 | 40 | 51 |
| 14 | Dextran AB (1,230) | 2 | 40 | 95 |
| 13 | Dextran AB (1,230) | 4 | 40 | 342 |
| 14 | Dextran AB (1,230) | 5 | 40 | 413 |
| 15 | Dextran AB (1,230) | 6 | 40 | 452 |
| 16 | Dextran AB (1,230) | 10 | 40 | 614 |

As can be seen from data in Table 3, severe Mw drop was noted even at the low 20kGy dose of gamma radiation. However a significant response to added wt% ascorbate versus molecular weight retention was observed notably around 4 wt%.

**Table 3. 20 kGy Experimental Results**

| Sample | Dextran source | Wt% ascorbate | Gamma dose (kGy) | Mw after irradiation |
|---|---|---|---|---|
| control | Dextran AB (1,230) | 0 | 20 | 21 |
| 10 | Dextran AB (1,230) | 5 | 20 | 586 |
| 11 | Dextran AB (1,230) | 6 | 20 | 616 |

At the lower dose of 20 kGy (Table 3) significantly less ascorbate was required to retain molecular weight.

Dextran having a Mw of greater than 200 kD is desirable for optimal sedimentation performance. Using combination of starting high molecular weight dextran in the presence of ascorbate achieves that goal. However due to the presence of ascorbate it was still desirable to confirm the cord blood sedimentation performance.

The extent of red blood cell aggregation was measured in vitro by mixing 5 mls cord blood (sourced from New York Blood Center) in a 20 ml tube (from Globe Scientific) with 10mls of aqueous dextran solutions from Table 4. A control sample was also prepared without the sodium ascorbate. The tube was capped and the contents mixed well by inverting the tube up and down. The contents were allowed to settle under gravity and the height of the RBC pellet was measured at 0,10,20,40, and 60 minutes.

As shown in Table 4 a number of the above samples were added to cord blood to assess the RBC sedimentation performance.

**Table 4. Sedimentation Performance**

| Sample # | Material | % Ascorbate | Gamma Dose (kGy) | MW after irradiation | | Aggregation (cm) | |
|---|---|---|---|---|---|---|---|
| | | | | | 0 min | 20 min | 60 min |
| 1 | Dextran | 0 | none | 1,230 | 12.2 | 3.0 | 2.4 |
| 2 | Dextran | 4 | none | 1,230 | 11.9 | 2.6 | 2.0 |
| 3 | Dextran | 0 | 40 | 35 | 12.0 | 11.8 | 11.5 |
| 4 | Dextran | 4 | 40 | 342 | 12.2 | 3.2 | 2.1 |
| 5 | Dextran | 5 | 40 | 413 | 12.0 | 3.5 | 2.0 |
| 6 | Dextran | 6 | 40 | 452 | 12.0 | 4.4 | 2.1 |

Table 4 shows that gamma irradiated dextran's in the presence of sodium ascorbate aggregates RBCs to similar extents as non-irradiated dextran's with and without sodium ascorbate.

## Claims

1. An aqueous dextran solution, stable to gamma irradiation comprising:
1 to 10 wt /v % of dextran where the dextran has an initial average molecular weight greater than 500kD;
2.0 to 20.0 wt% ascorbic acid or its mineral salt to the dextran.

2. The solution of claim 1 where the dextran has an initial molecular weight greater than 750kD.

3. The solution of claim 2 where the dextran has an initial molecular weight between approximately 1000 to 1500kD.

4. The solution of claim 1 where the mineral salt is sodium ascorbate.

5. The solution of claim 1 further comprising a buffer, a non-toxic enhancer or a combination thereof.

6. The solution of claim 5 where the non-toxic enhancer is sodium citrate, sodium succinate, or a combination thereof.

7. The solution of claim 5 where the buffer comprises organic or inorganic salts that maintain a pH of 4.0 to 8.0.

8. A kit for producing a gamma stabilized aqueous dextran solution comprising:
dextran where the dextran has an initial average molecular weight greater than 500kD; and
2.0 to 20.0 wt% ascorbic acid or its mineral salt to the dextran.

9. The kit of claim 8 where the dextran initial molecular weight is greater than 750kD.

10. The kit of claim 9 where the dextran initial molecular weight is between approximately 1000 to 1500kD.

11. The kit of claim 8 where the mineral salt is sodium asorbate.

12. The kit of claim 8 further comprising a buffer, a non-toxic enhancer or a combination thereof.

13. The kit of claim 12 where the non-toxic enhancer is sodium citrate, sodium succinate, or a combination thereof.

14. The kit of claim 12 where the buffer comprises organic or inorganic salts that maintain a pH of 4.0-9.0.

15. A method to aggregate cells in a sample comprising red blood cells (RBC), comprising the steps of:
a. obtaining an aqueous dextran solution, the aqueous dextran solution comprising;
1 to 10 wt/v % of dextran where the dextran has an initial average molecular weight greater than 500kD; and
2.0 to 20.0 wt% ascorbic acid or its mineral salt to the dextran;
b. exposing the solution to gamma radiation at a dose between 20 and 50 kGy; and
c. adding the sample to the aqueous dextran solution.

16. The method of claim 15 where the dextran has an initial molecular weight greater than 750kD.

17. The method of claim 16 where the dextran has an initial molecular weight between approximately 1000 to 1500kD.

18. The method of claim 15 where the mineral salt is sodium asorbate.

19. The method of claim 15 where the aqueous solution further comprises a buffer, a non-toxic enhancer or a combination thereof.

20. The method of claim 19 where the non-toxic enhancer is sodium citrate, sodium succinate, or a combination thereof.

21. The method of claim 19 where the buffer comprises organic or inorganic salts that maintain a pH of 4.0 to 8.0.

22. The method of claim 15 further comprising the steps of incubating the sample to aggregate and sediment of the red blood cells and optionally recovering total nucleated cells (TNC) from the sample.

23. The method of claim 22 were recovering the TNC comprises concentration of a liquid phase, said liquid phase comprising plasma and dextran using centrifugation, membrane filtration, or a combination thereof.

## Patentansprüche

1. Wässrige Dextranlösung, stabil gegenüber Gammabestrahlung, umfassend:
1 bis 10 Gew./V-% Dextran, wobei das Dextran ein anfängliches mittleres Molekulargewicht von über 500 kD aufweist;
2,0 bis 20,0 Gew.-% Ascorbinsäure oder ihr Mineralsalz zum Dextran.

2. Lösung nach Anspruch 1, wobei das Dextran ein anfängliches Molekulargewicht von über 750 kD aufweist.

3. Lösung nach Anspruch 2, wobei das Dextran ein anfängliches Molekulargewicht zwischen ungefähr 1000 und 1500 kD aufweist.

4. Lösung nach Anspruch 1, wobei das Mineralsalz Natriumascorbat ist.

5. Lösung nach Anspruch 1, ferner umfassend einen Puffer, einen nicht toxischen Verstärker oder eine Kombination davon.

6. Lösung nach Anspruch 5, wobei der nicht toxische Verstärker Natriumcitrat, Natriumsuccinat oder eine Kombination davon ist.

7. Lösung nach Anspruch 5, wobei der Puffer organische oder anorganische Salze umfasst, die einen pH von 4,0 bis 8,0 aufrechterhalten.

8. Kit zum Erzeugen einer gammastabilisierten, wässrigen Dextranlösung, umfassend:
Dextran, wobei das Dextran ein anfängliches mittleres Molekulargewicht von über 500 kD aufweist;
2,0 bis 20,0 Gew.-% Ascorbinsäure oder ihr Mineralsalz zum Dextran.

9. Kit nach Anspruch 8, wobei das Dextran ein anfängliches Molekulargewicht von über 750 kD aufweist.

10. Kit nach Anspruch 9, wobei das anfängliche Molekulargewicht des Dextrans zwischen ungefähr 1000 und 1500 kD liegt.

11. Kit nach Anspruch 8, wobei das Mineralsalz Natriumascorbat ist.

12. Kit nach Anspruch 8, ferner umfassend einen Puffer, einen nicht toxischen Verstärker oder eine Kombination davon.

13. Kit nach Anspruch 12, wobei der nicht toxische Verstärker Natriumcitrat, Natriumsuccinat oder eine Kombination davon ist.

14. Kit nach Anspruch 12, wobei der Puffer organische oder anorganische Salze umfasst, die einen pH von 4,0 bis 9,0 aufrechterhalten.

15. Verfahren zum Aggregieren von Zellen in einer Probe, die rote Blutkörperchen (RBC) umfassen, die folgenden Schritte umfassend:
a. Erhalten einer wässrigen Dextranlösung, die wässrige Dextranlösung umfassend:
1 bis 10 Gew./V-% Dextran, wobei das Dextran ein anfängliches mittleres Molekulargewicht von über 500 kD aufweist; und
2,0 bis 20,0 Gew.-% Ascorbinsäure oder ihr Mineralsalz zum Dextran;
b. b. Aussetzen der Lösung einer Gammabestrahlung mit einer Dosis zwischen 20 und 50 kGy; und
c. Zusetzen der Probe zur wässrigen Dextranlösung.

16. Verfahren nach Anspruch 15, wobei das Dextran ein anfängliches Molekulargewicht von über 750 kD aufweist.

17. Verfahren nach Anspruch 16, wobei das Dextran ein anfängliches Molekulargewicht zwischen ungefähr 1000 und 1500 kD aufweist.

18. Verfahren nach Anspruch 15, wobei das Mineralsalz Natriumascorbat ist.

19. Verfahren nach Anspruch 15, ferner umfassend einen Puffer, einen nicht toxischen Verstärker oder eine Kombination davon.

20. Verfahren nach Anspruch 19, wobei der nicht toxische Verstärker Natriumcitrat, Natriumsuccinat oder eine Kombination davon ist.

21. Verfahren nach Anspruch 19, wobei der Puffer organische oder anorganische Salze umfasst, die einen pH von 4,0 bis 8,0 aufrechterhalten.

22. Verfahren nach Anspruch 15, ferner umfassend die Schritte des Inkubierens der Probe zu Aggregat und Sediment der roten Blutkörperchen und des optionalen Rückgewinnens von völlig kernhaltigen Zellen (TNC) aus der Probe.

23. Verfahren nach Anspruch 22, wobei das Rückgewinnen der TNC Konzentration einer Flüssigphase, wobei die Flüssigphase Plasma und Dextran umfasst, unter Verwendung von Zentrifugation, Membranfilterung oder einer Kombination davon umfasst.

## Revendications

1. Solution aqueuse de dextrane stable aux rayons gamma comprenant :
1 à 10 % en poids/volume de dextrane où le dextrane a un poids moléculaire moyen initial supérieur à 500 kD ;
2,0 à 20,0 % en poids d'acide ascorbique ou de son sel minéral avec le dextrane.

2. Solution selon la revendication 1, dans laquelle le dextrane a un poids moléculaire initial supérieur à 750 kD.

3. Solution selon la revendication 2, dans laquelle le dextrane a un poids moléculaire initial entre environ 1000 et 1 500 kD.

4. Solution selon la revendication 1, dans laquelle le sel minéral est l'ascorbate de sodium.

5. Solution selon la revendication 1, comprenant en outre un tampon, un promoteur non toxique ou une de leurs combinaisons.

6. Solution selon la revendication 5, dans laquelle le promoteur non toxique est le citrate de sodium, le succinate de sodium ou une de leurs combinaisons.

7. Solution selon la revendication 5, dans laquelle le tampon comprend des sels organiques ou inorganiques qui maintiennent un pH de 4,0 à 8,0.

8. Kit de production d'une solution aqueuse de dextrane stabilisée aux rayons gamma comprenant :
du dextrane où le dextrane a un poids moléculaire moyen initial supérieur à 500 kD ; et
2,0 à 20,0 % en poids d'acide ascorbique ou de son sel minéral avec le dextrane.

9. Kit selon la revendication 8, dans lequel le poids moléculaire initial du dextrane est supérieur à 750 kD.

10. Kit selon la revendication 9, dans lequel le poids moléculaire initial du dextrane se situe entre environ 1 000 et 1 500 kD.

11. Kit selon la revendication 8, dans lequel le sel minéral est l'ascorbate de sodium.

12. Kit selon la revendication 8, comprenant en outre un tampon, un promoteur non toxique ou une de leurs combinaisons.

13. Kit selon la revendication 12, dans lequel le promoteur non toxique est le citrate de sodium, le succinate de sodium ou une de leurs combinaisons.

14. Kit selon la revendication 12, dans lequel le tampon comprend des sels organiques ou inorganiques qui maintiennent un pH de 4,0 à 9,0.

15. Procédé d'agrégation de cellules dans un échantillon comprenant des globules rouges (RBC), comprenant les étapes consistant à :
a. obtenir une solution aqueuse de dextrane, la solution aqueuse de dextrane comprenant :
1 à 10 % en poids/volume de dextrane où le dextane a un poids moléculaire moyen initial supérieur à 500 kD ; et
2,0 à 20,0 % en poids d'acide ascorbique ou de son sel minéral avec le dextrane ;
b. exposer la solution à des rayons gamma en dose comprise entre 20 et 50 kGy ; et
c. ajouter l'échantillon à la solution aqueuse de dextrane.

16. Procédé selon la revendication 15, dans lequel le dextrane a un poids moléculaire initial supérieur à 750 kD.

17. Procédé selon la revendication 16, dans lequel le dextrane a un poids moléculaire initial compris entre environ 1 000 et 1 500 kD.

18. Procédé selon la revendication 15, dans lequel le sel minéral est l'ascorbate de sodium.

19. Procédé selon la revendication 15, dans lequel la solution aqueuse comprend en outre un tampon, un promoteur non toxique ou une de leurs combinaisons.

20. Procédé selon la revendication 19, dans lequel le promoteur non toxique est le citrate de sodium, le succinate de sodium ou une de leurs combinaisons.

21. Procédé selon la revendication 19, dans lequel le tampon comprend des sels organiques ou inorganiques qui maintiennent un pH de 4,0 à 8,0.

22. Procédé selon la revendication 15, comprenant en outre les étapes d'incubation de l'échantillon pour agréger et sédimenter les globules rouges et de récupération éventuelle du total de cellules nucléées (TNC) de l'échantillon.

23. Procédé selon la revendication 22, dans lequel les TNC comprennent la concentration d'une phase liquide, ladite phase liquide comprenant du plasma et du dextrane, en utilisant une centrifugation, une filtration par membrane ou une de leurs combinaisons.
